# EUROPEAN PATENT APPLICATION

(11) **EP 4 082 434 A1**
(43) Date of publication of application: **02.11.2022**
(21) Application number: 21171015.7
(22) Date of filing: 28.04.2021
(51) Int. Cl.: A61B 5/08, A61B 5/097, B01J 20/28, A61B 10/00, G01N 30/12

(54) **AN AIR OR BREATH SAMPLING DEVICE CAPTURING BOTH AEROSOL AND VAPOR FRACTIONS**

(71) Applicant: Merck Patent GmbH, 64293 Darmstadt (DE)
(72) Inventor: NEUNER, Lisa, 64293 Darmstadt (DE); SEIPP, Charles, Milwaukee, WI-53209-3645 (US); BROWN, Jamie, Bellefonte, PA-16823-6217 (US)
(74) Representative: Merck Serono S.A. Intellectual Property

(57) **Abstract**

A sampling device for air or breath samples can collect both aerosols and particles as well as volatile organic compounds (VOC's) from the air or breath sample using a single device. The obtained samples are compatible with analytical methods to determine the presence or absence of (bio)markers without the need of an extensive work-up thereby simplifying their use and the analysis of air or breath samples.

## Description

### Field of the Invention.

The present invention relates to the collection of air or breath samples, such as breath samples from human breath, and a device which enables collecting both aerosols, particles and volatile organic compounds in a single device. The presence or absence of biomarkers in the collected air or breath samples can be determined from the samples collected with the device.

### Background.

Typically, breath capture devices focus solely on one breath fraction either volatile organic compounds (VOC), breath aerosols, or breath condensate. Each of these fractions contain unique information ranging from biomarkers, metabolites, organic compounds present in blood, or even particles. One severe limitation in the current state of the art is it is impossible to blow directly into a device for VOC analysis as back pressure is typically too great to facilitate this without mechanical mitigation methods (a pump, large dead volume, etc). Further, analysis of the amount of biomarker obtained is typically to be carried out without large amounts of time, resources, and expenses. Moreover, a large number of such markers can never be identified due to their complex molecular structure and/or to the amount of marker present in a sample collected with conventional methods.

More specifically, these days the field of breath sampling is rapidly expanding. A common approach for capturing breath samples is to have a person blow a breath sample into an air sampling bag followed by concentrating the contents of the sampling bag onto a thermal desorption tube that is packed with one or more adsorbents. Some sort of air sampling pump is required to pull the breath sample from the bag onto the thermal tube. These multiple steps are required because the backpressure of the typical thermal desorption tubes prevents a person from blowing directly into the TD tube. This backpressure is caused by the adsorbent particles and glass wool packed inside the tube.

In addition, the human breath contains both Aerosols and VOCs. For years most researchers have studied the VOC's fraction of the human breath. But there is a growing trend where researchers are now also exploring the aerosol fraction. The biomarkers of both fractions can provide researchers with additional biomarkers that are conclusively related to specific diseases or will advance future therapeutic drug discoveries. All of which will lead to the early detection of disease and how these diseases are treated in the future.

Accordingly, there is a need for a device which can collect the various (breath) fractions from an air or human breath sample in a single device capable of collecting aerosols, particles and volatile organic compounds (VOC's). Such device would need to have reduced backpressure or low or no backpressure to enable the collection of these fractions from an air or breath sample with the need for a pumping mechanism.

### Summary of the Invention.

The current invention solves the problems in collecting various fractions from air or breath samples described above providing a device which allows for the capture of two distinct air or breath fractions simultaneously-both aerosols and volatile organic compounds. Such device includes a filter part and an adsorbent part in the form of a honeycomb structure. This honeycomb structure, due to its air resistance-free packing, allows consumers or patients to blow directly into the device. This will allow for miniaturization and for the allowing the removal of the otherwise required presence of pumps or other electronic components. This results in a net reduction of complexity of the device for VOC analysis, while the filtered part allows for the collection of aerosols and particulate matter.

As such the current invention provides an air or breath sampling device with the ability to capture both fractions (aerosols and VOC's) during a single (breath) sample. The device is simple to use and does not require electricity or pumps. The collected fractions from the device could be easily analyzed for (bio)markers considering that the collected fractions are in a form which is compatible with current sample preparation workflows and can be analyzed using existing instrumentation. In addition, the device or the current invention allows a person to blow directly into a thermal desorption (TD) tube, which is in the form of a honeycomb structure, thus bypassing the need for the air sampling bag and air sampling pump. As such the use of the device of the present invention will simplify the process of capturing breath samples while also eliminating the hassles of transferring samples from bags to tubes. Moreover, the device of the present invention, while combining an aerosol or particle capturing means (e.g. a filtered part) and a VOC capturing means (e.g. a honeycomb structured part), has a low backpressure which enables a consumer or patient to easily blow into the device for collecting an air or breath sample.

### Brief Description of the Drawings.

Figure 1 shows a device comprising a means to collect aerosol and particles and a means to collect VOC's.
Figure 2 shows the results of a sample of the headspace of peppermint softgel from a 2-Liter static dilution bottle, i.e. the identification of the Volatile Peppermint Oil Components.
Figure 3 shows actual breath samples using both Aerosol Filter & Honeycomb TD Tube.
Figure 4 shows Volatile Compounds pass through the Filtered device.
Figure 5 shows an overview of a device according to the invention

### Detailed Description of the Invention.

As currently available air or breath sampling devices collect either an aerosol or a particulate fraction using a filtered device or device for adsorbing volatile organic compounds there is not a single device to collect both fractions in a single sampling method with the same single device. Typically, the means for adsorbing volatile organic compounds in a device creates backpressure which would then require a pumping mechanism to ensure airflow through the device. Combining such VOC collection means with a filtered device is problematic as it would be difficult to maintain proper airflow without a pumping mechanism. This would make is less desirable for use in for example collecting human breath samples.

The present invention overcomes these problems by combining a filtered collection means for collecting aerosols and particles with a honeycomb structured adsorbent collection means for collecting volatile organic compounds in a single air or breath sampling device. The combined device exhibits low back-pressure which allows a person to blow directly into the sampling device, eliminating the need to transfer the breath sample from a sampling bag, or the requirement of needing to pull the sample through the collection device with a vacuum or air sampling pump. As such a device according to the present invention does not suffer from the limitations caused by the backpressure of a VOC collection means. As a result, its use is simple for consumers to collect air or breath samples. It is relatively easy to breath into the device to collect breath samples for analysis of the obtained sample for the presence or absence of (bio)markers. In addition, the combined sampling device may incorporate a bag that inflates as indicator whether sufficient sample breaths have been collected.

As used herein the term volatile organic compound or VOC is meant to describe when relating to human breath sampling any organic chemical that can be emitted by the human body that is detectible in breath and/or constituents of the breath. When related to air sampling the term VOC can also include semi volatile compounds.

In addition, as used herein a breath biomarker or "breath analyte" is meant to describe a chemical component of breath or constituents of the breath that infers information on a state of the subject including but not limited to diagnostics, detection of illnesses, monitoring of disease progression, monitoring of organ rejection, environmental monitoring, exposure to toxins, radiation exposure, drug monitoring (both metabolized and unmetabolized molecules), pharmacokinetic and pharmacodynamic information, and metabolomics. With respect to air sampling the term marker refers to any compound (or combination of compounds) that can be used to indicate certain characteristics of the air, for example those related to air quality, or air quality fit for a certain purpose, or when the marker is below a certain threshold air safety (for example non-flammable or non-explosive).

In one embodiment of the present invention there is provided a device for collecting an air sample comprising: a.) a means for collecting aerosols and particles from ambient air, and b.) a means for collecting Volatile Organic Compounds (VOC's) from ambient air, wherein the means for collecting aerosols and particles and the means for collecting VOC's from ambient air are connected to each other such that the device collects from the same volume of ambient air aerosols, particles and VOC's.

In another embodiment of the present invention there is provided a device for capturing a breath sample from human breath comprising: a.) a means for collecting aerosols and particles from human breath, and b.) a means for collecting Volatile Organic Compounds (VOC's) from human breath, wherein the means for collecting aerosols and particles and the means for collecting VOC's from human breath are connected to each other such that the device collects from the same volume of exhaled human breath aerosols, particles and VOC's.

In such devices the means for collecting aerosols and particles comprises a filter to capture such aerosols and particles. Such filter can be an electrostatic filter.

It is important that the sampling of the air or breath sample is collected without the loss of sample during the collection process. Accordingly, in such air or breath sample collection device the means for collecting aerosols and particles and the means for collecting VOC's are connected through a gas-tight connection.

The volatile organic compounds are collected in the device through a means for collecting such VOC's which comprises a thermal desorption tube. Preferably such thermal desorption tube comprises a honeycomb structure. This honeycomb structure is coated with adsorbent material for capturing the VOC's. The adsorbent material can be a single type of adsorbent material or one or more different adsorbent materials to capture a broad range of organic molecules. Examples of such adsorbent materials includes active carbon, charcoal, silica gel(s), and porous polymers. Such collection means for the VOC's provide for a low backpressure ensuring air or breath sample without the need for a pumping mechanism. In addition, the collected samples could be readily analyzed using conventional analytical methods as the use of thermal desorption tubes is compatible with such analytical techniques such as various chromatographic techniques.

The honeycomb thermal desorption tube used in the device of the present invention can be constructed from any type of material which provide sufficient support for the adsorbent material and which can withstand temperatures of 450°C or more. In some embodiments the material can withstand temperatures of up to 1000°C.

In another embodiment of the present invention there is provided a method of sampling ambient air or human breath with the device according present invention.

In yet another embodiment of the present invention there is provided a method to determine the presence of a biomarker of interest in a sample of exhaled human breath or ambient air comprising; a.) collecting aerosols and particles from human breath or ambient air, b.) collecting Volatile Organic Compounds (VOC's) from human breath or ambient air, and c.) determining the presence of the biomarker of interest in either one of the collected aerosols, particles or VOC's, wherein the aerosols, particles and VOC's are collecting from the same exhaled human breath or same volume of ambient air and are collected in a single device according to the present invention.

In another embodiment of the present invention there is provided a method of diagnosing a patient for having or having a risk of having a disorder comprising determining the presence of a biomarker of interest comprising; a.) collecting aerosols and particles from human breath, b.) collecting Volatile Organic Compounds (VOC's) from human breath, c.) determining the presence of the biomarker of interest in either one of the collected aerosols, particles or VOC's, and d.) correlating the presence or absence of the biomarker of interest with a disease state, wherein the aerosols, particles and VOC's are collecting from the same exhaled human breath in a single device according to the present invention.

In each embodiment the device can be used either with or without, a means for collecting a specific subset of a breath phase such as the End tidal, mixed expiratory, or late expiratory breath. Any such means can include one of the following or any combination thereof: a flow meter (measuring flow or volume through either mechanical or electronic means), a sensor to detect concentrations of known/common constituents in breath (such as CO2, N2, fraction Nitric Oxide, or other), a timer-based method, humidity monitoring, or a rebreathing protocol.

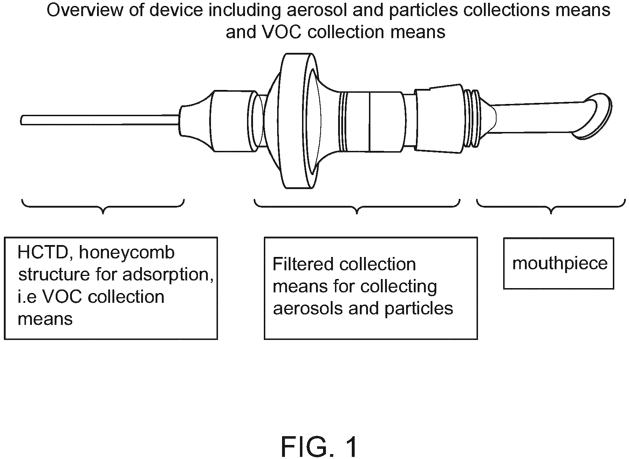

A schematic overview of an embodiment of an air or breath sampling device of the present invention is provided in Figure 1, which includes a VOC collection means for example in the form of a honeycomb structure including adsorbent(s), a collection means for aerosols and/or particles for example in the form of a filtered part, and a mouth piece for blow or receiving the air or breath from which the sampling is taken.

The following provides a description of exemplary embodiments of the present invention.

### Example 1. Use of a device capturing both aerosols and VOC's in a peppermint breath sampling protocol.

To evaluate a breath sampling device according to the present invention - breath samples were taken before and after ingesting peppermint softgel capsules (an OTC herbal supplement). During the ingestion process the components of the peppermint oil enter the blood stream where some the components migrate from the blood stream into the lungs where they are exhaled during normal breathing (Malásková, 2019). An experiment was performed to demonstrate how this new breath sampling device could be used to capture the volatile components of the metabolized peppermint oil capsules.

The device uses a ceramic honeycomb that is coated with a carbon adsorbent. The honeycomb structure exhibits minimal backpressure thus allowing a person to easily blow a breath sample into the tube.

The honeycomb thermal desorption tubes (HCTD Tube) were constructed by coating two separate ceramic honeycombs with two different adsorbents with increasing adsorptive strength. The first honeycomb was coated with Carbopack^{™} C, and the second honeycomb was coated with Carbopack B. The coating process took place by suspending the nano-size carbon adsorbents particles in a solution containing methylene chloride and polysilazane which serves as a "glue" to adhere the carbon adsorbents to the ceramic honeycombs. The ceramic honeycombs were dipped into the solution that coated all of the exposed surfaces of the ceramic honeycomb. Multiple coats were applied until a build-up was observed. The coated honeycombs were then placed in a Stainless Steel thermal desorption tube 6.35mm OD x 5mm ID x 89mm long. The coated assemblies (tubes) were placed in a glass purge bomb and heated to 450°C for 10min while Nitrogen gas passed through to create an inert atmosphere while the curing process occurred.

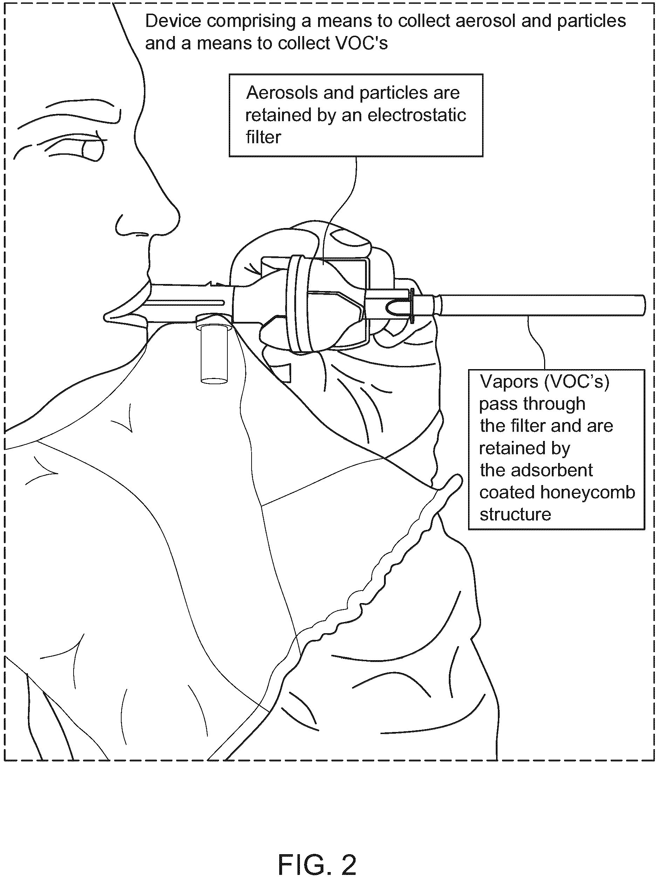

The Breath Sampling device according to the present invention was created by coupling an aerosol and particle collecting device containing a filter, such as the SensAbues Device, with the Honeycomb Thermal Desorption tube (HCTD tube) as prepared as described above (figure 2). To combine these two devices the outlet storage cap of the filtering device was drilled out so the inlet of the HCTD tube could be inserted into the cap. Then the HCTD tube was connected to the filtered device.

The focus of this experiment was focused on whether breath sample could be collected with and without the electrostatic filtering device coupled with the honeycomb tubes.

The primary purpose of connecting the two devices is to capture both the aerosol and the vapor fraction of human breath with a single breath sample. When a person exhales into the coupled device, the aerosols along with any particles will be retained by the electrostatic filter in the device whereas the volatile compounds in the gas phase will pass through the filter and then be retained by the adsorbent coated HCTD tubes. Volatiles that are entrapped within the aerosols that are then released upon impaction with the filter will be subsequently be retained by the HCTD tube as the person continues to breathe into the device. After taking the breath sample each sampling device is individually sealed to protect the sample integrity until they are analyzed.

There is an additional benefit to coupling a filtering device with the Honeycomb Thermal Desorption tube. The filter protects the thermal desorption tube from retaining non-volatile compounds during breath sampling. Non volatiles are not amendable to GC Analysis. This prevents the non-volatiles from fouling up the TD/GC sample pathway during thermal desorption.

### Example 2; taking a breath sample with the device.

Protocol used for the Breath Sampling Experiment
1. Fasted overnight (no breakfast)
2. Brushed teeth with baking soda (to prevent contamination caused by using mint flavored toothpaste)
3. Rinse mouth twice with bottled water - Prior to taking breath samples
4. Capture a baseline breath sample before ingesting the peppermint capsules
5. Consume 5qty peppermint capsules wait 1-hour to metabolize the capsules
6. Collect breath sample using our prototype breath sampling device.

For this of experiment: One blew into the combined breath sampling device for 20qty breaths, this took approx. 6 minutes and 30 second to complete. The filtered device uses a small bag as an indicator, so users know when they have blown enough air through the device. When using the filtered device by itself the bag is full after approx. 20 breaths. The minor backpressure of combining the two devices, i.e. the filtered device together with the honeycomb structure, together doesn't cause any problems of blowing a breath sample into the device.

For this experiment the following combinations of breath samples were captured:
1. Before and after ingesting the peppermint capsules.
2. Samples with and without the filtered device connected to the HCTD Tubes
3. Sample with Uncoated Honeycombs & Adsorbent Coated HCTD Tubes Before taking breath samples the HCTD Tubes were conditioned at 330°C for 30 minutes to reduce the volatile background levels using CDS Analytical 6-tube conditioner. The filtered device was used as they were received. The devices were inside a sealed bag contained in a cardboard box.

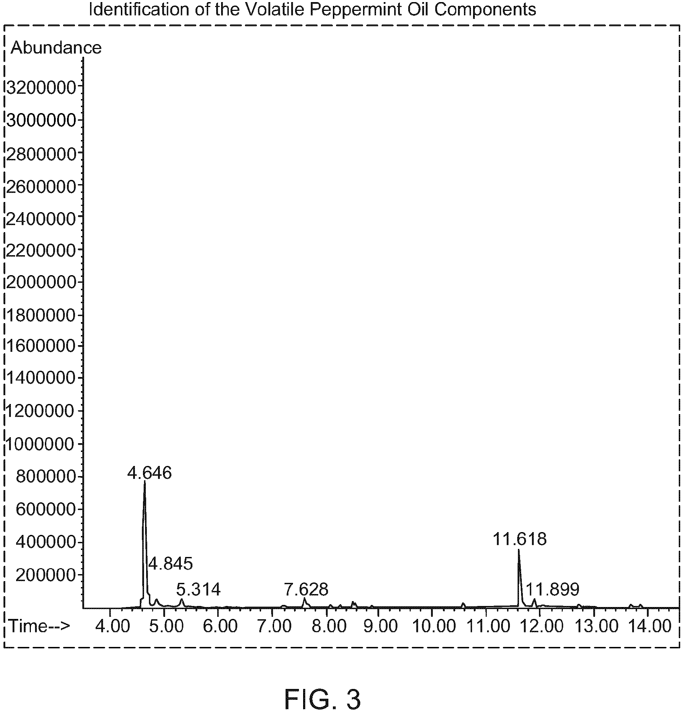

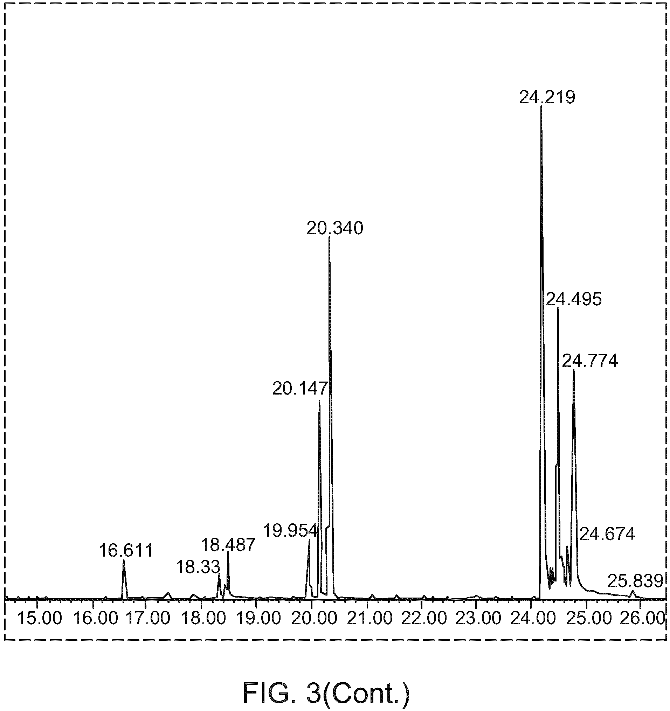

Figure 3 shows the results of a sample of the headspace of peppermint softgel from the 2-Liter static dilution bottle. This was obtained by injecting 1mL of the headspace onto a traditional thermal desorption tube that was packed Carbopack C and Carbopack B. The identification of the components was based on matches to the NIST mass spec library. Individual chemical standards of each component were not obtained so quantification and identification did not occur.

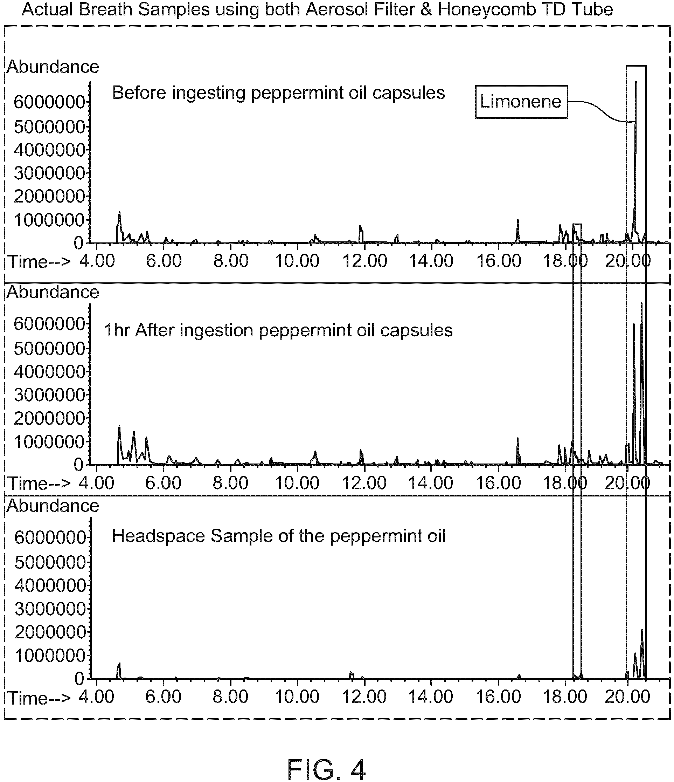

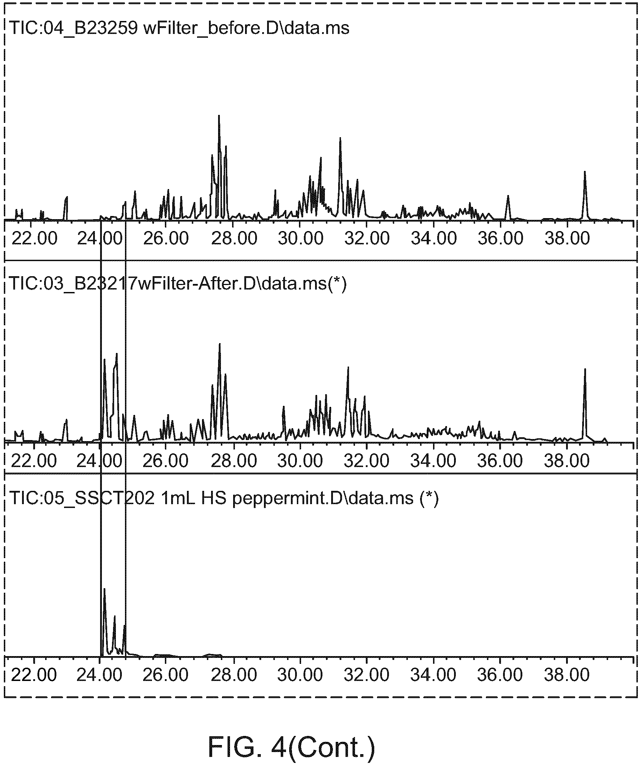

Figure 4 shows an overlay chromatogram of breath Samples with the filtered device in series with the HCTD Tube. These results are of the HCTD Tube not of the filter. The top chromatogram (black) is the breath sample prior to ingesting the peppermint capsules. The middle chromatogram (blue) is the analysis of the HCTD Tube 1-hour after ingesting the peppermint capsules. The bottom chromatogram (red) is of a headspace sample of the peppermint oil obtained from the 2-liter static dilution bottle. A syringe volume of 750µL of the headspace of peppermint oil was injected onto a traditional thermal desorption tube packed with Carbopack C and Carbopack B particles.

The highlighted peaks in blue are where the peppermint oil components eluted. Of the eleven identified components of the peppermint oil only Limonene was observed from the breath sample prior ingesting the peppermint oil capsules. It turns out Limonene is a chemical typically observed in human breath and maybe produced by the liver and expelled by the breath. The major takeaway is the majority of the peppermint components were observed 1-hour after consuming the peppermint oil capsules (blue chromatogram) demonstrating how the said device can retain VOC's from human breath.

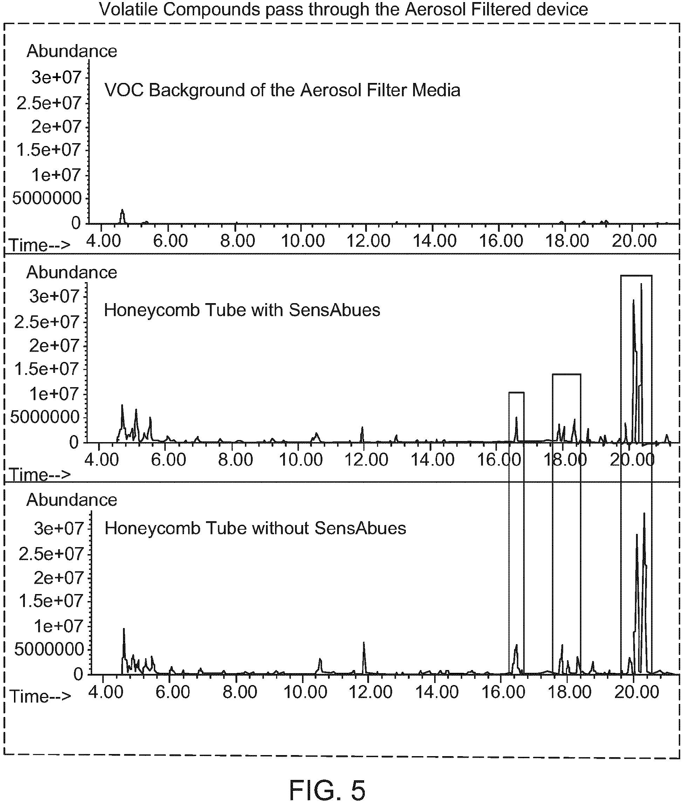

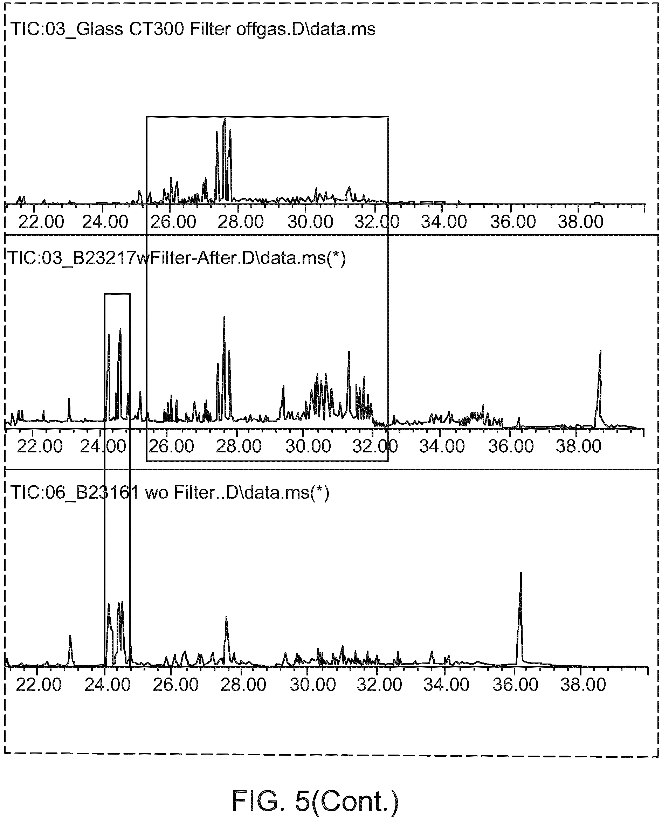

Figure 5 shows a chromatogram overlay that shows how the HCTD tube captured the peppermint oil components with and without the filtered device attached (the middle and bottom chromatogram). It is noted that the HCTD tube with the filtered attached had more volatiles retained than when blown into the HCTD tube without the filtered device. It turns out the filter of the filtered device does have some volatile contaminates (see the red highlighted area). However, this graphic shows that the volatile peppermint oil components showed similar results with and without the filtered device. This provides evidence that the volatile peppermint oil components don't get retained by the filter of the filtered device.

These examples demonstrate that the breath sampling device can capture volatile compounds from human breath. These examples also demonstrate that the volatile compound pass through the filtered device part and are collected on the HCTD Tube.

This air or breath sampling device of the present invention eliminates the need to transfer samples from air sampling bags on to tubes. This is a significant advancement since it would allow for a more standardized breath sampling protocol to be used when analyzing air or breath samples for determining the presence or absence of (bio)markers.

## Claims

1. A device for collecting an air sample comprising:
a. a means for collecting aerosols and particles from ambient air, and
b. a means for collecting Volatile Organic Compounds (VOC's) from ambient air,
wherein the means for collecting aerosols and particles and the means for collecting VOC's from ambient air are connected to each other such that the device collects from the same volume of ambient air aerosols, particles and VOC's.

2. A device for capturing a breath sample from human breath comprising:
a. a means for collecting aerosols and particles from human breath, and
b. a means for collecting Volatile Organic Compounds (VOC's) from human breath,
wherein the means for collecting aerosols and particles and the means for collecting VOC's from human breath are connected to each other such that the device collects from the same volume of exhaled human breath aerosols, particles and VOC's.

3. The device according to claim 1 or 2, wherein the means for collecting aerosols and particles comprises a filter to capture such aerosols and particles.

4. The device according to claim 3, wherein the filter is an electrostatic filter.

5. The device of any of the preceding claims, wherein the means for collecting aerosols and particles and the means for collecting VOC's are connected through a gas-tight connection.

6. The device according to any of the preceding claims, wherein the means for collecting VOC's comprises a thermal desorption tube.

7. The device according to claim 6, wherein the thermal desorption tube comprises a honeycomb structure.

8. The device according to claim 7, wherein the honeycomb structure is made from any material which is capable of resisting temperatures of 450°C or more.

9. The device according to claim 7, wherein the honeycomb structure comprises adsorbent material.

10. The device according to claim 9, wherein the honeycomb structure is coated with one or more adsorbent materials.

11. A method of sampling ambient air with the device according to any of the preceding claims.

12. A method of sampling human breath with the device according to any of claims 2 to 10.

13. A method to determine the presence of a biomarker of interest in a sample of exhaled human breath or ambient air comprising
a. collecting aerosols and particles from human breath or ambient air,
b. collecting Volatile Organic Compounds (VOC's) from human breath or ambient air, and
c. determining the presence of the biomarker of interest in either one of the collected aerosols, particles or VOC's,
wherein the aerosols, particles and VOC's are collecting from the same exhaled human breath or same volume of ambient air and are collected in a single device according to any of claims 1 to 10.

14. A method of diagnosing a patient for having or having a risk of having a disorder comprising determining the presence of a biomarker of interest comprising
a. collecting aerosols and particles from human breath,
b. collecting Volatile Organic Compounds (VOC's) from human breath,
c. determining the presence of the biomarker of interest in either one of the collected aerosols, particles or VOC's, and
d. correlating the presence or absence of the biomarker of interest with a disease state,
wherein the aerosols, particles and VOC's are collecting from the same exhaled human breath in a single device according to any of claims 2 to 10.
